# NEW EUROPEAN PATENT SPECIFICATION

(11) **EP 2 135 599 B2**
(45) Date of publication and mention of the opposition decision: **25.05.2016**
(45) Mention of the grant of the patent: 22.02.2012
(21) Application number: 08010879.8
(22) Date of filing: 16.06.2008
(51) Int. Cl.: A61K 8/34, A61K 8/39, A61Q 5/08

(54) **Decolouring composition for keratin fibres**
Entfärbungszusammensetzung für Keratinfasern
Composition de décoloration pour fibres de kératine

(43) Date of publication of application: 23.12.2009
(73) Proprietor: Kao Germany GmbH, 64297 Darmstadt (DE)
(72) Inventor: Uellner, Martin, Mr., 64295 Darmstadt (DE); Hullmann, Alexandra, Dr., 64331 Weiterstadt (DE); Dürr, Manfred, 64839 Münster (DE)
(74) Representative: Michalski Hüttermann & Partner Patentanwälte mbB

(56) References cited:
- EP-A1- 1 036 558
- EP-A1- 1 086 685
- DE-A1- 4 402 328
- DE-A1- 10 118 176
- DE-C1- 19 737 987
- FR-A1- 2 833 836
- JP-A- 11 335 243
- JP-A- 2001 206 839
- JP-A- 2007 131 569
- US-A- 3 616 803

## Description

Present invention relates to a decolouring composition for keratin fibres especially human hair. Particularly, the present invention relates to a decolouring composition for hair coloured with direct dyes and especially with anionic direct dyes.

Permanent and semi-permanent hair colouring has been widely used. One of the difficulties in hair dyeing process is that in case of unsatisfactory colour result at the end of a colouring process, correction is quite difficult as it includes removing only artificial colour from hair which is not an easy task. In removing permanent colours, which are also known as oxidative colours, reducing agent comprising compositions are often used.

In order to remove semi-permanent colours obtained with direct dyes, various solutions have been used, however, unsuccessfully. Extend of success of removal of semi-permanent colours is very much dependent on the depth of penetration of the dyes into the hair fibres during dyeing process.

Within the scope of the present description, semi-permanent dyes include in particular hair dyeing with direct dyes such as cationic, anionic and non-ionic nitro dyes, especially with anionic direct dyes and especially in an acidic pH range.

Present invention aims at providing a composition for effectively decolouring keratin fibres, especially human hair, which is coloured with a colouring composition comprising at least one hair direct dye such as cationic, anionic and non-ionic nitro dyes, and especially comprising at least one anionic direct dye and has an acidic pH.

Present inventors have surprisingly found out that an aqueous composition comprising
o water at a concentration between 40 and 70% by weight, calculated to total composition,
o at least one polyol at a concentration of 10 to 30% by weight, calculated to total composition,
o at least one C₂-C₆ aliphatic alcohol,
o at least one thickening agent selected from non-ionic cellulose derivatives
   and having
o a viscosity value between 1000 and 40 000 mPa.s. measured at 20°C with Brookfield viscosimeter at 5 rpm with a spindle 5 and a pH in the range of 8 to 12
   removes artificial colour from keratin fibres, especially human hair, coloured with direct dyes such as cationic, anionic and non-ionic nitro dyes and especially with anionic direct dyes.

Accordingly, the first object of the present invention is defined in independent claim 1.

In the preferred embodiment of the present invention, aqueous decolouring composition further comprises at least one aromatic alcohol.

It is further object of the present invention that above mentioned composition is for decolouring keratin fibres, especially human hair, that is coloured with a composition comprising at least one direct dye and has a pH between 1 and 4.

Further object of the present invention is that above mentioned composition is for decolouring hair that is coloured with a composition comprising at least one anionic direct dye and has a pH between 1 and 4.

Decolouring composition of the present invention comprises between 45 and 70%, more preferably 50 and 65% and most preferably 50 and 60%, by weight, calculated to total composition.

Decolouring compositions or the present invention comprises at least one polyol at a concentration of 10 to 30%, preferably 10 to 25% and more preferably 15 to 25% by weight, calculated to total composition. In the preferred embodiment of the present invention at least one polyol is a butylene glycol or a compound according to the general formula 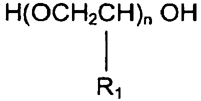 wherein n has a value between 1 and 75 and R₁ is H or CH₃.

Suitable polyols are propylene glycols such as propylene glycol, dipropyleneglycol, PPG-3, PPG-7, PPG-9, PPG-12, PPG-13, PPG-15, PPG-16, PPG-17, PPG-20, PPG-26, PPG-30, PPG-33, PPG-34, PPG-51 and PPG-69, ethylene glycols such as PEG-4, PEG-6, PEG-7, PEG-8, PEG-9, PEG-10, PEG-12, PEG-14, PEG-16, PEG-18, PEG-20, PEG-32, PEG-33, PEG-40, PEG-45, PEG-45, PEG-60 and PEG-75, butylene glycols such as butylene glycol, 1,2-butylene glycol, 1,3-butylene glycol, and 2,3-butylene glycol. Preferred are propylene glycol, dipropyleneglycol, PPG-3, PPG-7, PPG-9, PPG-12, PPG-13, PPG-15, PPG-16, PPG-17, PPG-20, PEG-4, PEG-6, PEG-7, PEG-8, PEG-9, PEG-10, PEG-12, PEG-14, PEG-16, PEG-18, PEG-20, butylene glycol, 1,2-butylene glycol, 1,3-butylene glycol, and 2,3-butylene glycol. More preferred are propylene glycol, dipropyleneglycol, PPG-3, PPG-7, PPG-9, PPG-12, PPG-13, PPG-15, PPG-16, PPG-17, PPG-20, PEG-4, PEG-6, PEG-7, PEG-8, PEG-9, PEG-10, PEG-12, PEG-14, PEG-16, butylene glycol, 1,2-butylene glycol, 1,3-butylene glycol, and 2,3-butylene glycol. Most preferred ones are propylene glycol, dipropyleneglycol, PPG-3, PPG-7, PPG-9, PEG-6, PEG-7, PEG-8, PEG-9, PEG-10, PEG-12, butylene glycol, 1,2-butylene glycol, 1,3-butylene glycol, and 2,3-butylene glycol.

In further preferred embodiment of the present invention decolouring composition comprises at least one (poyl) propylene glycol, at least one polyethylene glycol and at least one butylene glycol. The above mentioned concentration ranges for polyol are valid as the total polyol concentration in the compositions. Particularly, the weight ratio of at least one (poyl)propylene glycol, at least one polyethylene glycol and at least one butylene glycol is in the range of 1:0.2:0.2 - 1:1:1, more preferably in the range of 1:0.4:0.4 - 1:0.8:0.8, and most preferably 1:0.5:0.5 - 1:0.8:0.8.

Decolouring compositions comprise at least one C₂-C₆ aliphatic alcohol at a concentration of 5 to 40%, preferably 10 to 35% more preferably 10 to 30% and most preferably 15 to 25% by weight calculated to total composition.

Suitable aliphatic alcohols are ethanol, propanol, iso-propanol, n-butanol, pentanol and hexanol. Preferred are ethanol, propanol and iso propanol and more preferred are ethanol and propanol and most preferred is ethanol.

pH of the compositions is between 8 and 12, preferably 8.5 to 11, more preferably 9 to 11 and most preferably 10 to 11. pH of the compositions are adjusted with any water soluble alkalizing agent however preferred ethanolamine and its derivatives and the most preferred is monoethanolamine.

In a further preferred embodiment of the present invention decolouring composition comprises at least one aromatic alcohol according to the general formula 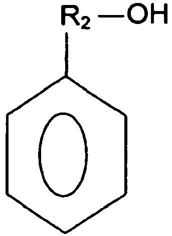 wherein R₂ is a straight or branched alkyl chain with 1 to 6 C atoms or a CH₂O CH₂ CH₂ group.

Non-limiting suitable examples to aromatic alcohol are benzyl alcohol, benzyl carbinol, methyl phenylbutanol and benzyl glycol. Preferred are benzyl alcohol and benzyl carbinol, most preferred is benzyl alcohol.

At least one aromatioc alcohol is comprised in decolouring compositions of present invention at a concentration of 1 to 20%, preferably 2 to 15%, more preferably 2 to 10% and most preferably 3 to 7.5% by weight calculated to total composition.

In a further embodiment of the present invention, decolouring composition comprises at least one cationic surfactant presented with the general formula 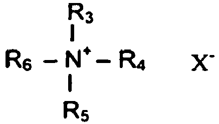 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has value of 1 - 4, or

R₈ CO O (CH₂)ₙ

where R₈ is saturated or unsaturated, branched or non-branched alkyl chain with 7-21 C atoms and n has value of 1 - 4, and R₄ is hydrogen or unsaturated or saturated, branched or non-branched alkyl chain with 1 - 22 C atoms or

R₇ CO NH (CH₂)ₙ

or

R₈ CO O (CH₂)ₙ

where R₇, R₈ and n are same as above.

R₅ and R₆ are hydrogen or lower alkyl chain with 1 to 4 carbon atoms, and X is anion such as chloride, bromide, methosulfate, as a conditioning agent.

Preferably, cationic surfactant as conditioning agent includes only one long chain alkyl group and, therefore, R₄ is hydrogen or an alkyl chain with 1 to 4 C atoms. Typical suitable examples of cationic surfactants are cetyl trimethly ammonium chloride, steartrimonium chloride, behentrimoinium chloride, stearamidopropyl trimonuim chloride, dioleoylethyl dimethyl ammonium methosulfate, dioleoylethyl hydroxyethylmonium methosulfate. Preferred are cetyl trimethly ammonium chloride, steartrimonium chloride, behentrimoinium chloride.

Concentration of the cationic surfactants in the composition is between 0.01 and 10%, preferably, 0.1 and 7.5 and most preferably 0.25 and 5% by weight calculated to total composition.

Aqueous decolouring composition of the present invention further comprises a cationic polymer as a conditioning agent. Suitable ones are those of known with their CTFA name Polyquaternium. Some examples are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquatemium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquatemium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquatemium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87. As well those polymers known with their CTFA category name Quaternium are suitable. Those are for example Quaternium-8, Quatemium-14, Quaternium-15, Quaternium-18, Quatemium-22, Quaternium-24, Quaternium-26, Quatemium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

It has further been found out that especially those of cationic cellulose type polymers known as Polymer JR type from Amerchol such as Polyquaternium 10 or cationic galactomannans such as cationic guar gum known with trade name Jaguar from Rhone-Poulenc which are chemically for example Guar hydroxypropyl trimonium chloride and cationic tara gum an its derivatives known with INCI name Caesalpinia spinosa hydroxypropyltrimonium chloride, are preferred ones which act at the same time as a thickener which may further be combined with a preferred non-ionic polymer. Furthermore, chitosan and chitin can also be included in the compositions as cationic natural polymers. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 67 and polyquaternium 70.

The cationic polymers also include the quaternized products of graft polymers from organopolysiloxanes and polyethyl oxazolines described in EP-A 524 612 and EP-A 640 643.

Conditioning cationic polymers are included into to the compositions in the range of 0.1 to 5% by weight, calculated to total composition

In order to ease the application and achieve longer contact time between keratin fibres especially human hair and decolouring composition, the claimed composition is thickened with a thickening agent to a viscosity value between 1000 and 40000 mPa.s, measured at 20°C with Brookfiled viscoismetre with an appropriate spindle, spindle 5, at 5 rpm. Viscosity is preferably 5000 to 30000 and more preferably 7500 to 25000 and most preferably 10000 to 25000 mPa.s measured as mentioned above.

Accordingly, composition of the present invention comprises at least one thickening agent selected from cellulose derivatives such as hydroxyethylcellulose, hydroxybutyl methylcellulose, hydroxyethyl ethylcellulose, hydroxypropyl cellulose, hydroxypropyl methylcellulose, methylcellulose, methyl ethylcellulose and methyl hydroxyethylcellulose. Most preferred is hydroxypropyl methylcellulose. Concentration of thickening polymer should be adjusted according to the required viscosity of the composition, and typically be in the range of 0.1 to 5% by weight, calculated to total composition.

Decolouring composition can further comprise surfactants especially non-ionic and amphoteric ones, fragrances, chelating agents, natural and/or synthetic oils and acidifying compounds to increase or built up a buffer capacity.

Present invention is as well as on a process for decolouring hair wherein a composition disclosed above is applied onto coloured hair especially coloured with an anionic direct dye and kept on the hair for a period of 5 to 30 min at ambient temperature to 40°C and rinsed off from hair, optionally hair is shampooed and optionally coloured again.

Following examples are to illustrate the invention but not to limit it.

### Example 1

| | % by weight |
|---|---|
| Ethanol | 20.0 |
| Propylene glycol | 10.0 |
| Benzyl alcohol | 5.0 |
| PEG-8 | 5.0 |
| Butylene glycol | 5.0 |
| Hydroxypropyl methylcellulose | 1.2 |
| Cetrimonium chloride | 2.0 |
| Monoethanolamine | q.s. to pH 10.5 |
| Fragrance | 0.5 |
| Water | to 100 |

Above composition has a viscosity of 15000 mPa.s measured at 20°C with a Brookfield viscosimetre using spindle 5.

Decolouring composition of example 1 was applied onto a hair tress coloured with commercially available colouring product under the brand name Elumen, shade NB@5, an processed for 30 min at ambient temperature. The natural hair colour was medium blonde at level 8. The tress was washed with tap water and shampooed once and dried. Colour intensity was measured with laboratory equipment. The following results were obtained.

| | L values |
|---|---|
| Before colouring | 42.8 |
| After colouring with NB@5 | 26.2 |
| After decolouring | 38.9 |

L value is commonly used to present colour intensity of human hair. The higher the value, the lighter the colour. The above results show clearly that original hair colour is largely recovered after application of decolouring composition of Example 1.

Similar results were obtained with following examples.

### Example 2

| | % by weight |
|---|---|
| Propanol | 20.0 |
| Propylene glycol | 15.0 |
| PEG-10 | 5.0 |
| 1,3-Butylene glycol | 5.0 |
| Hydroxypropylcellulose | 1.2 |
| Steartrimonium chloride | 2.0 |
| Monoethanolamine | q.s. to pH 10.2 |
| Fragrance | 1.0 |
| Water | to 100 |

### Example 3

| | % by weight |
|---|---|
| Ethanol | 15.0 |
| PPG-3 | 20.0 |
| Benzyl carbinol | 5.0 |
| PEG-6 | 5.0 |
| 2,3-Butylene glycol | 5.0 |
| Hydroxyethyl ethylcellulose | 1.5 |
| Cetrimonium chloride | 2.0 |
| Monoethanolamine | q.s. to pH 10.8 |
| Fragrance | 0.5 |
| Water | to 100 |

### Example 4

| | % by weight |
|---|---|
| Ethanol | 20.0 |
| Propylene glycol | 10.0 |
| Benzyl alcohol | 5.0 |
| PEG-8 | 5.0 |
| Butylene glycol | 5.0 |
| Hydroxypropyl methylcellulose | 1.2 |
| Cetrimonium chloride | 2.0 |
| Monoethanolamine | q.s. to pH 10.5 |
| Fragrance | 0.5 |
| Water | to 100 |

### Example 5

| | % by weight |
|---|---|
| Ethanol | 25.0 |
| Dipropylene glycol | 15.0 |
| PEG-10 | 5.0 |
| Hydroxypropyl methylcellulose | 1.8 |

| | % by weight |
|---|---|
| Stearamidopropyltrimonium chloride | 2.0 |
| Monoethanolamine | q.s. to pH 11 |
| Fragrance | 0.8 |
| Water | to 100 |

### Example 6

| | % by weight |
|---|---|
| Ethanol | 20.0 |
| Propylene glycol | 10.0 |
| Benzyl alcohol | 5.0 |
| PEG-8 | 5.0 |
| Butylene glycol | 5.0 |
| Hydroxypropyl methylcellulose | 0.8 |
| Polymer JR 400 | 0.5 |
| Cetrimonium chloride | 1.2 |
| Monoethanolamine | q.s. to pH 10.3 |
| Fragrance | 0.5 |
| Water | to 100 |

## Claims

1. Aqueous decolouring composition for keratin fibres, especially human hair, coloured with a composition comprising at least one direct dye **characterised in that** it comprises water at a concentration between 40 and 70% by weight, calculated to total composition, at least one polyol at a concentration of 10 to 30% by weight, calculated to total composition, at least one C₂-C₆ aliphatic alcohol, at least one thickening agent selected from non-ionic cellulose derivatives and has a viscosity value between 1000 and 40000 mPa.s. measured at 20°C with Brookfield viscosimetre at 5 rpm with a spindle 5 and having a pH in the range of 8 to 12.

2. Composition according to claim 1 **characterised in that** keratin fibres, especially human hair is coloured with a composition comprising at least one anionic direct dye.

3. Composition according to any of the preceding claims **characterised in that** polyol is selected from a butylene glycol and compounds according to general formula 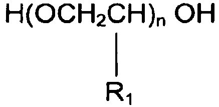 wherein n has a value between 1 and 75 and R₁ is H or CH₃.

4. Composition according to any of the preceding claims **characterised in that** it comprises at least one (poly)propyleneglycol, at least one polyethyleneglycol and at least one butylene glycol in a weight ratio of (poly)propyleneglycol : polyethyleneglycol : a butylene glycol in the range of 1:0.2:0.2 to 1:1:1.

5. Composition according to any of the preceding claims **characterised in that** it comprises at least one C₂ - C₆ aliphatic alcohol at a concentration of 5 to 40% by weight, calculated to total composition.

6. Composition according to any of the preceding claims **characterised in that** it comprises at least one aromatic alcohol according to general formula 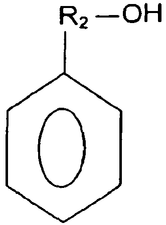 wherein R₂ is a straight or branched alkyl chain with 1 to 6 C atoms or a CH₂O-CH₂-CH₂ group.

7. Composition according to claim 6 **characterised in that** aromatic alcohol is selected from benzyl alcohol, benzyl carbinol, methyl phenylbutanol and benzyl glycol, and especially is benzyl alcohol.

8. Composition according to any of the preceding claims **characterised in that** it comprises at least one cationic surfactant according to general formula 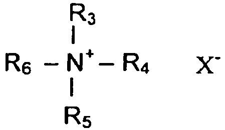 where R₃ is a saturated or unsaturated, branched or non-branched alkyl chain with 8-22 C atoms or
R₇-CO-NH-(CH₂)ₙ
where R₇ is saturated or unsaturated, branched or non-branched alkyl chain with 7-21 C atoms and n has value of 1 - 4, or
R₈-CO-O-(CH₂)ₙ
where R₈ is saturated or unsaturated, branched or non-branched alkyl chain with 7-21 C atoms and n has value of 1 - 4, and
R₄ is hydrogen or unsaturated or saturated, branched or non-branched alkyl chain with 1 - 22 C atoms or
R₇-CO-NH-(CH₂)ₙ
or
R⁸-CO-O-(CH₂)ₙ
where R₇, R₈ and n are same as above,
R₅ and R₆ are hydrogen or lower alkyl chain with 1 to 4 carbon atoms, and X is anion such as chloride, bromide, methosulfate.

9. Composition according to any of the preceding claims **characterised in that** it comprises at least one cationic polymer.

10. Composition according to any of the preceding claims **characterised in that** at least one thickening agent selected from cellulose derivatives is selected from hydroxyethylcellulose, hydroxybutyl methylcellulose, hydroxyethyl ethyl cellulose, hydroxypropyl cellulose, hydroxypropyl methylcellulose, methylcellulose, methyl ethylcellulose and methyl hydroxyethylcellulose.

11. Use of composition according to any of the preceding claims for decolouring keratin fibres, especially human hair, coloured with a composition comprising at least one direct dye, especially anionic direct dye and having acidic pH.

12. Process for decolouring keratin fibres especially human hair wherein a composition according to claims 1 to 10 is applied onto coloured hair especially freshly coloured hair and processed for 5 to 45 min at temperatures between ambient temperature and 40°C and rinsed off and optionally shampooed and optionally dried or left to dry in air.

## Patentansprüche

1. Wässrige Entfärbungszusammensetzung für Keratinfasern, insbesondere menschliches Haar, welches mit einer Zusammensetzung gefärbt ist, die mindestens einen Direktfarbstoff aufweist, **dadurch gekennzeichnet, dass** sie Wasser in einer Konzentration von 40 bis 70 Gewichts-%, bezogen auf die Gesamtzusammensetzung, mindestens ein Polyol in einer Konzentration von 10 bis 30 Gewichts-%, bezogen auf die Gesamtzusammensetzung, mindestens einen aliphatischen C₂₋₆-Alkohol, mindestens ein Verdickungsmittel, ausgewählt aus nichtionischen Cellulosederivaten, aufweist und einen Viskositätswert von 1.000 bis 40.000 mPa·s aufweist, gemessen bei 20 °C mit einem Brookfield-Viskosimeter bei 5 U/min mit einer Spindel 5, und einen pH-Wert im Bereich von 8 bis 12 aufweist.

2. Zusammensetzung nach Anspruch 1, **dadurch gekennzeichnet, dass** die Keratinfasern, insbesondere das menschliche Haar, mit einer Zusammensetzung gefärbt sind, welche mindestens einen anionischen Direktfarbstoff aufweist.

3. Zusammensetzung nach einem der vorhergehenden Ansprüche, **dadurch gekennzeichnet, dass** das Polyol ausgewählt ist aus einem Butylenglykol und Verbindungen der allgemeinen Formel 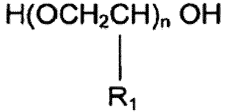 wobei n einen Wert von 1 bis 75 aufweist und R₁ für H oder CH₃ steht.

4. Zusammensetzung nach einem der vorhergehenden Ansprüche, **dadurch gekennzeichnet, dass** sie mindestens ein (Poly)propylenglykol, mindestens ein Polyethylenglykol und mindestens ein Butylenglykol in einem Gewichtsverhältnis (Poly)propylenglykol : Polyethylenglykol : Butylenglykol im Bereich von 1:0,2:0,2 bis 1:1:1 aufweist.

5. Zusammensetzung nach einem der vorhergehenden Ansprüche, **dadurch gekennzeichnet, dass** sie mindestens einen aliphatischen C₂₋₆-Alkohol in einer Konzentration von 5 bis 40 Gewichts-% aufweist, bezogen auf die Gesamtzusammensetzung.

6. Zusammensetzung nach einem der vorhergehenden Ansprüche, **dadurch gekennzeichnet, dass** sie mindestens einen aromatischen Alkohol der allgemeinen Formel 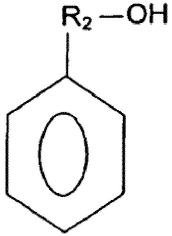 aufweist, wobei R₂ für eine lineare oder verzweigte Alkylkette mit 1 bis 6 C-Atomen oder eine CH₂O-CH₂-CH₂-Gruppe steht.

7. Zusammensetzung nach Anspruch 6, **dadurch gekennzeichnet, dass** der aromatische Alkohol aus Benzylalkohol, Benzylcarbinol, Methylphenylbutanol und Benzylglykol ausgewählt ist und insbesondere Benzylalkohol ist.

8. Zusammensetzung nach einem der vorhergehenden Ansprüche, **dadurch gekennzeichnet, dass** sie mindestens ein kationisches Tensid der allgemeinen Formel 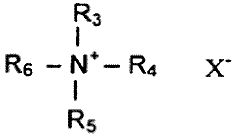 aufweist, wobei R3 für eine gesättigte oder ungesättigte, verzweigte oder unverzweigte Alkylkette mit 8 bis 22 C-Atomen oder
R₇-CO-NH-(CH₂)ₙ
wobei R₇ für eine gesättigte oder ungesättigte, verzweigte oder unverzweigte Alkylkette mit 7 bis 21 C-Atomen steht und n einen Wert von 1 bis 4 aufweist, oder
R₈-CO-O-(CH₂)ₙ
steht, wobei R₈ für eine gesättigte oder ungesättigte, verzweigte oder unverzweigte Alkylkette mit 7 bis 21 C-Atomen steht und n einen Wert von 1 bis 4 aufweist, und R₄ für Wasserstoff oder eine gesättigte oder ungesättigte, verzweigte oder unverzweigte Alkylkette mit 1 bis 22 C-Atomen oder
R₇-CO-NH-(CH₂)ₙ
oder
R₈-CO-O-(CH₂)ₙ
steht, wobei R₇, R₈ und n für dasselbe wie oben stehen,
R₅ und R₆ für Wasserstoff oder eine niedere Alkylkette mit 1 bis 4 Kohlenstoffatomen steht und X für ein Anion wie Chlorid, Bromid, Methosulfat steht.

9. Zusammensetzung nach einem der vorhergehenden Ansprüche, **dadurch gekennzeichnet, dass** sie mindestens ein kationisches Polymer aufweist.

10. Zusammensetzung nach einem der vorhergehenden Ansprüche, **dadurch gekennzeichnet, dass** das mindestens eine aus Cellulosederivaten ausgewählte Verdickungsmittel aus Hydroxyethylcellulose, Hydroxybutylmethylcellulose, Hydroxyethylethylcellulose, Hydroxypropylcellulose, Hydroxypropylmethylcellulose, Methylcellulose, Methylethylcellulose und Methylhydroxyethylcellulose ausgewählt ist.

11. Verwendung einer Zusammensetzung nach einem der vorhergehenden Ansprüche zum Entfärben von Keratinfasern, insbesondere menschlichem Haar, welches mit einer Zusammensetzung gefärbt ist, die mindestens einen Direktfarbstoff aufweist, insbesondere einen anionischen Direktfarbstoff, und einen sauren pH-Wert aufweist.

12. Verfahren zum Entfärben von Keratinfasern, insbesondere menschlichem Haar, wobei eine Zusammensetzung nach Anspruch 1 bis 10 auf gefärbtes Haar, insbesondere frisch gefärbtes Haar, aufgebracht wird und bei Temperaturen von Umgebungstemperatur bis 40 °C 5 min bis 45 min lang einwirkt und ausgespült wird und gegebenenfalls shampooniert und gegebenenfalls getrocknet oder zum Trocknen an der Luft gelassen wird.

## Revendications

1. Composition aqueuse de décoloration pour fibres de kératine, notamment des cheveux humains colorés avec une composition comprenant au moins un colorant direct, **caractérisé en ce qu'**elle comprend de l'eau à une concentration entre 40 et 70 % en poids, calculée par rapport à la composition totale, au moins un polyol à une concentration de 10 à 30 % en poids, calculée par rapport à la composition totale, au moins un alcool aliphatique en C₂-C₆, au moins un agent épaississant choisi parmi des dérivés non ioniques de la cellulose et a une valeur de viscosité entre 1000 et 40000 mPa mesurée à 20 °C avec un viscosimètre Brookfield à 5 tours/minute avec une broche de 5 et ayant un pH dans une gamme de 8 à 12.

2. Composition selon la revendication 1, **caractérisée en ce que** les fibres de kératine, particulièrement les cheveux humains, sont colorés avec une composition comprenant au moins un colorant direct anionique.

3. Composition selon l'une quelconque des revendications précédentes, **caractérisée en ce que** le polyol est choisi parmi un butylène glycol et des composés de formule générale 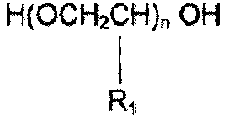 dans laquelle n prend une valeur entre 1 et 75 et R₁ est un atome H ou un groupe CH₃.

4. Composition selon l'une quelconque des revendications précédentes, **caractérisée en ce qu'**elle comprend au moins un (poly)propylène glycol, au moins un polyéthylène glycol et au moins un butylène glycol dans un ratio pondéral de (poly)propylène glycol : polyéthylène glycol : un butylène glycol dans la gamme de 1 : 0,2 : 0,2 à 1 : 1 : 1.

5. Composition selon l'une quelconque des revendications précédentes, **caractérisée en ce qu'**elle comprend au moins un alcool aliphatique en C₂-C₆ à une concentration de 5 à 40 % en poids, calculée par rapport à la composition totale.

6. Composition selon l'une quelconque des revendications précédentes, **caractérisée en ce qu'**elle comprend au moins un alcool aromatique selon la formule générale 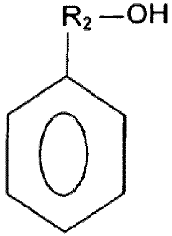 dans laquelle R₂ est une chaîne aliphatique linéaire ou ramifiée, avec 1 à 6 atomes de C ou un groupe CH₂O-CH₂-CH₂.

7. Composition selon la revendication 6, **caractérisée en ce que** l'alcool aromatique est choisi parmi l'alcool benzylique, le benzyl carbinol, le méthyl phénylbutanol et le benzyl glycol, et est particulièrement de l'alcool benzylique.

8. Composition selon l'une quelconque des revendications précédentes, **caractérisée en ce qu'**elle comprend au moins un tensio-actif cationique selon la formule générale 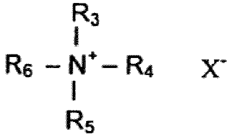 où R₃ est une chaîne alkyle saturée ou non saturée, linéaire ou ramifiée, de 8 à 22 atomes de C ou
R₇-CO-NH-(CH₂)ₙ
où R₇ est une chaîne alkyle saturée ou non saturée, linéaire ou ramifiée, de 7 à 21 atomes de C et n prend une valeur entre 1 et 4, ou
R₈-CO-O-(CH₂)ₙ
où R₈ est une chaîne saturée ou non saturée, linéaire ou ramifiée, de 7 à 21 atomes de C et n prend une valeur entre 1 et 4, et
R₄ est un atome d'hydrogène ou une chaîne saturée ou non saturée, linéaire ou ramifiée, de 1 à 22 atomes de C ou
R₇-CO-NH-(CH₂)ₙ
ou
R₈-CO-O-(CH₂)ₙ
où R₇, R₈ and n sont identiques à ci-dessus,
R₅ et R₆ sont des atomes d'hydrogène ou une chaîne alkyle plus courte de 1 à 4 atomes de carbone, et X est un anion tel que le chlorure, le bromure, le méthosulfate.

9. Composition selon l'une quelconque des revendications précédentes, **caractérisée en ce qu'**elle comprend au moins un polymère cationique.

10. Composition selon l'une quelconque des revendications précédentes, **caractérisée en ce qu'**au moins un agent épaississant choisi parmi des dérivés de la cellulose est choisi parmi l'hydroxyéthylcellulose, l'hydroxybutyl méthylcellulose, l'hydroxyéthyl éthyl cellulose, l'hydroxypropyl cellulose, l'hydroxypropyl méthylcellulose, la méthylcellulose, la méthyl éthylcellulose et la méthyl hydroxyéthylcellulose.

11. Utilisation de la composition selon l'une quelconque des revendications précédentes pour la décoloration des fibres de kératine, particulièrement des cheveux humains, colorés par une composition comprenant au moins un colorant direct, particulièrement un colorant direct anionique qui a un pH acide.

12. Procédé de décoloration des fibres de kératine, particulièrement des cheveux humains, où la composition selon les revendications 1 à 10 est appliquée sur des cheveux colorés, en particulier sur des cheveux fraîchement colorés, et que l'on laisse agir pendant 5 à 45 minutes à une température entre la température ambiante et 40°C et qui sont rincés et éventuellement shampouinés et éventuellement séchés ou laissés sécher à l'air.
